# EUROPEAN PATENT APPLICATION

(11) **EP 4 023 209 A1**
(43) Date of publication of application: **06.07.2022**
(21) Application number: 21804328.9
(22) Date of filing: 02.04.2021
(51) Int. Cl.: A61K 8/9794, A61K 8/9789, A61K 8/81, A61K 8/73, A61K 8/44, A61K 8/39, A61K 8/36, A61Q 19/10, A61Q 19/00, A61P 37/08, A61P 29/00

(54) **ANTI-DRY AND ANTI-SENSITIVITY COMPOSITION AND PREPARATION METHOD THEREFOR**

(30) Priority: 15.05.2020 CN 202010411182
(71) Applicant: Guangzhou Keneng Cosmetics Research Co. Ltd, Guangzhou, Guangdong 510800 (CN); Guangdong Danz Group Co., Ltd., Guangzhou, Guangdong 510000 (CN)
(72) Inventor: YUAN, Jingbo, Guangzhou, Guangdong 510800 (CN); LI, Chuanmao, Guangzhou, Guangdong 510800 (CN); LIN, Shengjie, Guangzhou, Guangdong 510800 (CN); ZHANG, Chubiao, Guangzhou, Guangdong 510800 (CN); ZENG, Weidan, Guangzhou, Guangdong 510800 (CN); ZHANG, Weijie, Guangzhou, Guangdong 510800 (CN)
(74) Representative: AWA Sweden AB
(86) International application number: PCT/CN2021/085223
(87) International publication number: WO 2021/227700

(57) **Abstract**

An anti-dry and anti-sensitivity composition and a preparation method therefor. The composition comprises the following components by the total mass: 10-40% of an anionic surfactant, 1-12% of a nonionic surfactant, 1-28% of a zwitterionic surfactant, 0.5-40% of a plant extract composition, 0.5-35% of grease, and 0.1-6% of a skin conditioning agent. The amino acid surfactants are rationally matched, thus forming a mild and foam-rich amino acid combined framework, having a milder cleaning function compared with traditional surfactants, and avoiding skin dryness due to excessive degreasing; in addition, the plant extract composition is matched, achieving multiple functions of anti-sensitivity, anti-inflammation and moisturizing on the skin, and various components are combined, enabling a synergistic effect in a shower gel. The composition can have multiple effects of mild cleaning, moisturizing, anti-sensitivity and the like, and have no irritation to the skin, and is safe and mild. Further, the preparation method for a shower gel composition has high safety and relatively low cost, and is suitable for large-scale industrial production.

## Description

### Technical Field

The present disclosure belongs to the technical field of cosmetics, and particularly relates to an anti-dry and anti-sensitivity composition and a preparation method thereof.

### Background Art

With the improvement of living standards, the frequency of use of shower gel has increased. However, due to the influence of various factors such as improper selection, frequent use, and improper use of shower gel, and skin problems, some people will have skin problems such as peeling, itching, and sensitivity.

There are many kinds of shower gels on the market currently, but there are very few shower gels specifically developed for people having sensitive, dry, or itchy skin, or problematic skin with their own defects. The skin needs to be gently cleaned, and meanwhile, requires multiple effects such as a moisturizing and nourishing effect, an anti-sensitivity effect, and an anti-inflammatory effect. Therefore, it is very important to develop a shower gel suitable for this kind of people.

### Summary of the Invention

In one aspect, the present disclosure provides a plant extract composition, including an *Ulmus davidiana* extract, a *Glycyrrhiza uralensis* extract, a *Citrus paradisi* extract, a *Brasenia schreberi* extract, and a *Phyllostachys bambusoides* juice extract, which are purchased from a cosmetic raw material supplier (HYUNDAI BIOLAND, South Korea). The plant extract composition includes the following components in percentage by weight: 0.1-30% of *Ulmus davidiana* extract, 0.05-45% of *Glycyrrhiza uralensis* extract, 0.01-26% of *Citrus paradisi* extract, 0.05-20% of *Brasenia schreberi* extract, and 0.02-35% of *Phyllostachys bambusoides* juice extract.

The *Ulmus davidiana* extract is extracted from root bark of *Ulmus davidiana,* which contain rich natural polysaccharides such as galactose, rhamnose, glucose, glucosamine, and trehalose. It can inhibit the hyaluronidase activity and reduce transepidermal water loss, and has strong moisturizing and anti-aging effects. Furthermore, the *Ulmus davidiana* extract can also repair damage caused by UVA irradiation and improve the cell viability, and meanwhile, has a good anti-inflammatory effect.

The *Glycyrrhiza uralensis* extract contains glycyrrhizic acid serving as main ingredients, which can effectively scavenge free radicals and inhibit the lipoxygenase activity, thereby having anti-aging and anti-inflammatory effects.

The *Citrus paradisi* extract has a broad-spectrum anti-bacterial effect, and can inhibit growth of microbial strains such as *Escherichia coli, Staphylococcus aureus, Bacillus subtilis,* and *Candida albicans.*

The *Brasenia schreberi* extract is an agar-like mucus secreted on the underside of watershield leaf, contains rich saccharides, can form a layer of protective film on the skin to enable the skin to lock more moisture therein and increase moisture retention of the skin, and has anti-pollution and antioxidant capacities.

The *Phyllostachys bambusoides* juice extract has skin-beautifying vitality, which can moisturize the skin and enhance the skin barrier.

In one aspect, the present disclosure provides a composition, including the following components in percentage by weight: 10-40% of anionic surfactant, 1-12% of nonionic surfactant, 1-28% of zwitterionic surfactant, 0.5-40% of the plant extract composition, 0.5-35% of grease, and 0.1-6% of skin conditioning agent.

In some embodiments, the anionic surfactant is selected from at least one of sodium lauroyl sarcosinate, sodium lauroyl alaninate, sodium lauroyl glutamate, sodium lauroyl oat amino acids, sodium cocoyl apple amino acids, and sodium taurine cocoyl methyltaurate.

In some embodiments, the anionic surfactant is selected from the following components in percentage by weight: 3-30%, and preferably 6-20% of sodium lauroyl sarcosinate; 0.5-10%, and preferably 1-7% of sodium lauroyl alaninate; 0.1-9%, and preferably 0.5-6% of sodium lauroyl glutamate; 0.2-11%, and preferably 0.5-8% of sodium lauroyl oat amino acids; 0.5-12%, and preferably 1-5% of sodium cocoyl apple amino acids; and 1-12%, and preferably 3-8% of sodium taurine cocoyl methyltaurate.

In some embodiments, the nonionic surfactant is selected from at least one of cocamide MEA and cocamide methyl MEA.

In some embodiments, the nonionic surfactant is selected from the following components in percentage by weight: 0.1-5%, and preferably 0.5-2% of cocamide MEA; and 0.2-6%, and preferably 0.6-3% of cocamide methyl MEA.

In some embodiments, the zwitterionic surfactant is selected from at least one of cocamidopropyl betaine, sodium lauroamphoacetate, and lauryl hydroxysultaine.

In some embodiments, the zwitterionic surfactant is selected from the following components in percentage by weight: 0.5-25%, and preferably 1-20% of cocamidopropyl betaine; 0.3-13%, and preferably 0.4-12% of sodium lauroamphoacetate; and 0.1-23%, and preferably 0.3-9% of lauryl hydroxysultaine.

In some embodiments, the plant extract composition is selected from the following components in percentage by weight: 0.1-20%, and preferably 0.5-15% of *Ulmus davidiana* extract; 0.1-16%, and preferably 0.3-15% of *Glycyrrhiza uralensis* extract; 0.2-8%, and preferably 0.5-6% of *Citrus paradisi* extract; 0.03-17%, and preferably 0.1-11% of *Brasenia schreberi* extract, and 0.03-5%, and preferably 0.2-4% of *Phyllostachys bambusoides* juice extract.

In some embodiments, the grease is selected from at least one of PEG-7 glyceryl cocoate, isostearic acid, and glyceryl laurate.

In some embodiments, the grease is selected from the following components in percentage by weight: 0.1-4%, and preferably 0.2-1% of PEG-7 glyceryl cocoate; 0.1-2%, and preferably 0.2-1% of isostearic acid; and 0.1-5%, and preferably 0.2-3% of glyceryl laurate.

In some embodiments, the skin conditioning agent is selected from at least one of polyquaternium-7 and polyquaternium-10.

In some embodiments, the skin conditioning agent is selected from the following components in percentage by weight: 0.05-1%, and preferably 0.1-0.6% of polyquaternium-7; and 0.01-2%, and preferably 0.1-0.6% of polyquaternium-10.

In some embodiments, the composition further includes one or more of a preservative, a chelator, a pH regulator, a fragrance, and water.

In some embodiments, the preservative is selected from at least one of phenoxyethanol, ethylhexylglycerin, and p-hydroxyacetophenone.

In some embodiments, the preservative is selected from the following components in percentage by weight: 0.2-1%, and preferably 0.3-0.6% of phenoxyethanol; 0.1-1%, and preferably 0.2-0.6% of ethylhexylglycerin; and 0.1-1%, and preferably 0.2-0.5% of p-hydroxyacetophenone.

In some embodiments, the chelator is selected from EDTA-2Na; and preferably, the chelator has a weight percentage of 0.1-0.6%, and preferably 0.2-0.4%.

In some embodiments, the pH regulator is selected from at least one of citric acid, sodium citrate, aminomethyl propanol, and triethanolamine.

In some embodiments, the pH regulator is selected from citric acid; and further preferably, the citric acid has a weight percentage of 0.01-8%.

In some embodiments, the fragrance has a weight percentage of 0.02%.

In one aspect, the present disclosure also provides a preparation method of the composition, characterized by including the following steps:
at step 1), adding an anionic surfactant and water into a reaction kettle, stirring, and heating to 75-80°C;
at step 2), adding a zwitterionic surfactant, and stirring uniformly;
at step 3), further adding a nonionic surfactant and a chelator, stirring uniformly, keeping the temperature for 20-30 min, and cooling;
at step 4), cooling to 45°C, adding a plant composition, a preservative, and grease, and stirring uniformly;
at step 5), adding a skin conditioning agent and a fragrance, and regulating pH to 5.5-7.3; and
at step 6), cooling to 37°C, and filtering.

The present disclosure achieves the following beneficial effects.

The combination of various plant extracts of the present disclosure has a moisturizing and nourishing effect, an anti-sensitivity effect, an anti- anti-inflammatory effect, etc. When added to cosmetics, these extracts can achieve an anti-dry effect, and also can achieve repairing skin barrier, soothing and anti-sensitivity, antioxidation effects, etc. By use of amino acid surfactants and addition of grease and skin conditioning agent, a mild and foam-rich amino acid composition framework is formed. A shower gel of the present disclosure achieves a synergistic effect through the combination of various components.

Further, tests prove that the composition of the present disclosure has no irritation to human body, and the preparation method of a shower gel composition of the present disclosure has high safety and relatively low cost, and is suitable for large-scale industrial production.

### Detailed Description of the Invention

The technical solutions of the present disclosure will be further described below with reference to specific embodiments, but the scope of protection of the present disclosure is not limited thereto. All non-essential modifications and adjustments made by others according to the concept of the present disclosure shall fall within the scope of protection of the present disclosure.

Examples 1 to 3 Shower gels containing the composition of the present disclosure Various components of the shower gels were prepared according to Table 1.

**Table 1 Formulas of shower gels of Examples 1 to 3**

| | | Example 1 | Example 2 | Example 3 |
|---|---|---|---|---|
| Anionic surfactant | Sodium lauroyl sarcosinate | 7 | 8 | 8 |
| | Sodium lauroyl alaninate | 2 | 1.5 | 1.5 |
| | Sodium lauroyl glutamate | 1 | 0.8 | 0.8 |
| | Sodium lauroyl oat amino acids | 0.6 | 0.7 | 0.7 |
| | Sodium cocoyl apple amino acids | 1.5 | 2 | 2 |
| | Sodium taurine cocoyl methyltaurate | 4 | 4.5 | 4.5 |
| Nonionic surfactant | Cocamide MEA | 1 | 1.5 | 1.5 |
| | Cocamide methyl MEA | 0.7 | 0.6 | 0.6 |
| Zwitterionic surfactant | Cocamidopropyl betaine | 1.5 | 2 | 2 |
| | Sodium lauroamphoacetate | 0.5 | 0.5 | 0.5 |
| | Lauryl hydroxysultaine | 0.4 | 0.4 | 0.4 |
| Plant extract composition | *Ulmus davidiana* extract | 0.7 | 1 | 1 |
| | *Glycyrrhiza uralensis* extract | 0.5 | 1.5 | 1.5 |
| | *Citrus paradisi* extract | 1 | 0.7 | 0.7 |
| | *Brasenia schreberi* extract | 0.2 | 0.5 | 0.5 |
| | *Phyllostachys bambusoides* juice extract | 0.3 | 0.5 | 0.5 |
| Grease | PEG-7 glyceryl cocoate | 0.5 | 0.5 | 0.5 |
| | Isostearic acid | 0.2 | 0.3 | 0.3 |
| | Glyceryl laurate | 0.5 | 0.5 | 0.5 |
| Skin conditioning agent | Polyquaternium-7 | 0.2 | 0.15 | 0.15 |
| | Polyquaternium-10 | 0.1 | 0.1 | 0.1 |
| Preservative | Phenoxyethanol | 0.5 | 0.5 | 0.5 |
| | Ethylhexylglycerin | 0.3 | 0.3 | 0.3 |
| | P-hydroxyacetophenone | 0.3 | 0.3 | 0.3 |
| Fragrance | | 0.02 | 0.02 | 0.02 |
| Chelator | EDTA-2Na | 0.2 | 0.2 | 0.2 |
| pH regulator | | q.s. | q.s. | q.s. |
| Water | | to 100 | to 100 | to 100 |

The shower gels of Examples 1 to 3 were prepared by the following method:
at step ①, sodium lauroyl sarcosinate, sodium lauroyl alaninate, sodium lauroyl glutamate, sodium lauroyl oat amino acids, sodium cocoyl apple amino acids, sodium taurine cocoyl methyltaurate, and water were added into a reaction kettle, and the mixture was stirred uniformly and heated to 75-80°C;
at step ②, cocamidopropyl betaine, sodium lauroamphoacetate, and lauryl hydroxysultaine were added, and the mixture was stirred uniformly;
at step ③, cocamide MEA, cocamide methyl MEA, and EDTA-2Na were added, the mixture was stirred uniformly, the temperature was kept for 20-30 min and then reduced;
at step ④, the mixture was cooled to 65°C, and p-hydroxyacetophenone was added;
at step ⑤, the mixture was cooled to 45°C, an *Ulmus davidiana* extract, a *Glycyrrhiza uralensis* extract, a *Citrus paradisi* extract, a *Brasenia schreberi* extract, a *Phyllostachys bambusoides* juice extract, PEG-7 glyceryl cocoate, isostearic acid, glyceryl laurate, phenoxyethanol, and ethylhexylglycerin were added, and the mixture was stirred uniformly;
at step ⑥, polyquaternium-7, polyquaternium-10, and a fragrance were added, and pH was regulated with citric acid and sodium citrate to 5.5-7.3;
at step ⑦, the mixture was cooled to 37°C, filtered, and discharged; and
at step ⑧, after passing inspection, the mixture was divided, packaged, and inspected again, and finished products were stored in a warehouse.

### Comparative Examples 1 to 13

Various components of shower gels were prepared according to Table 2.

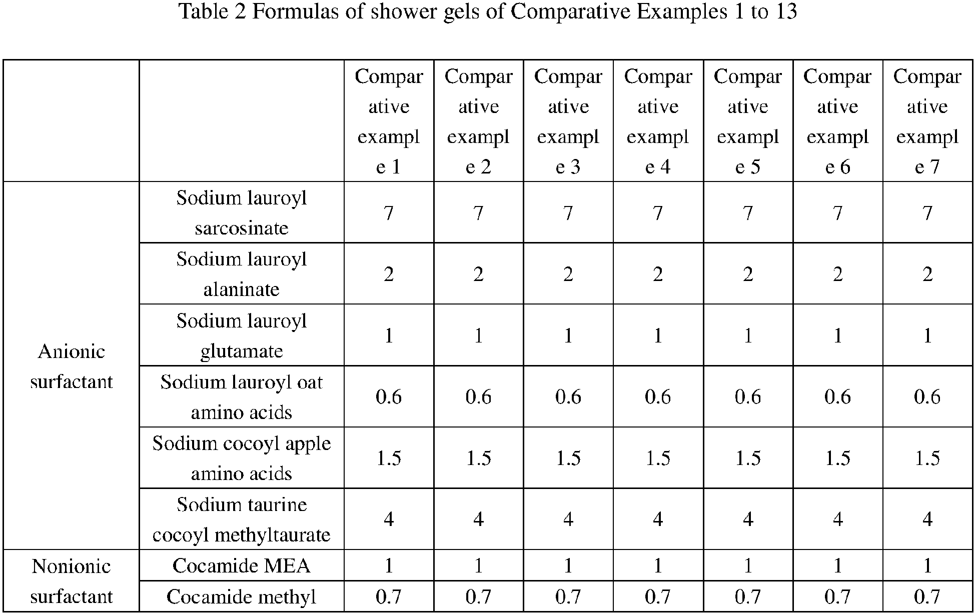

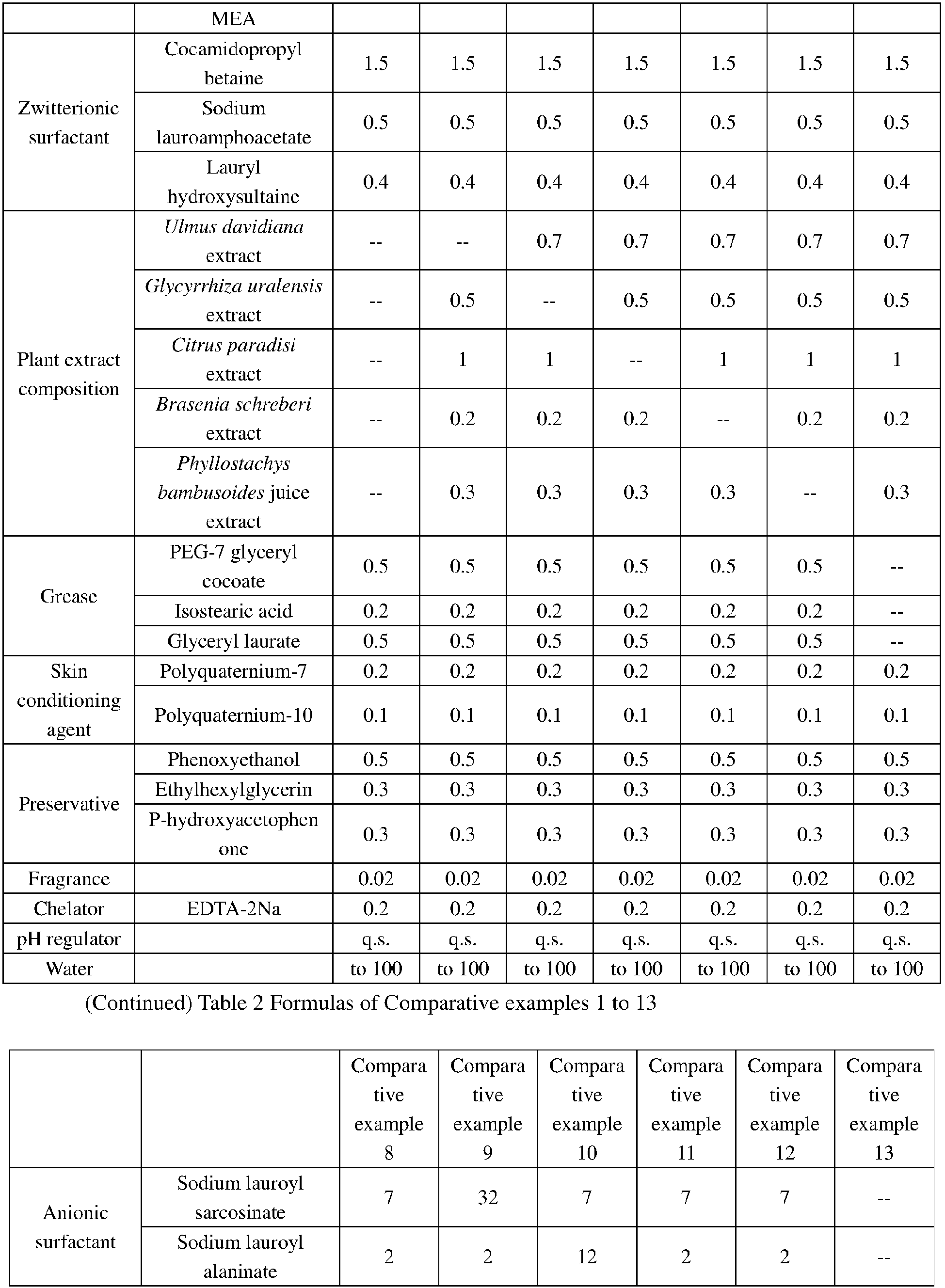

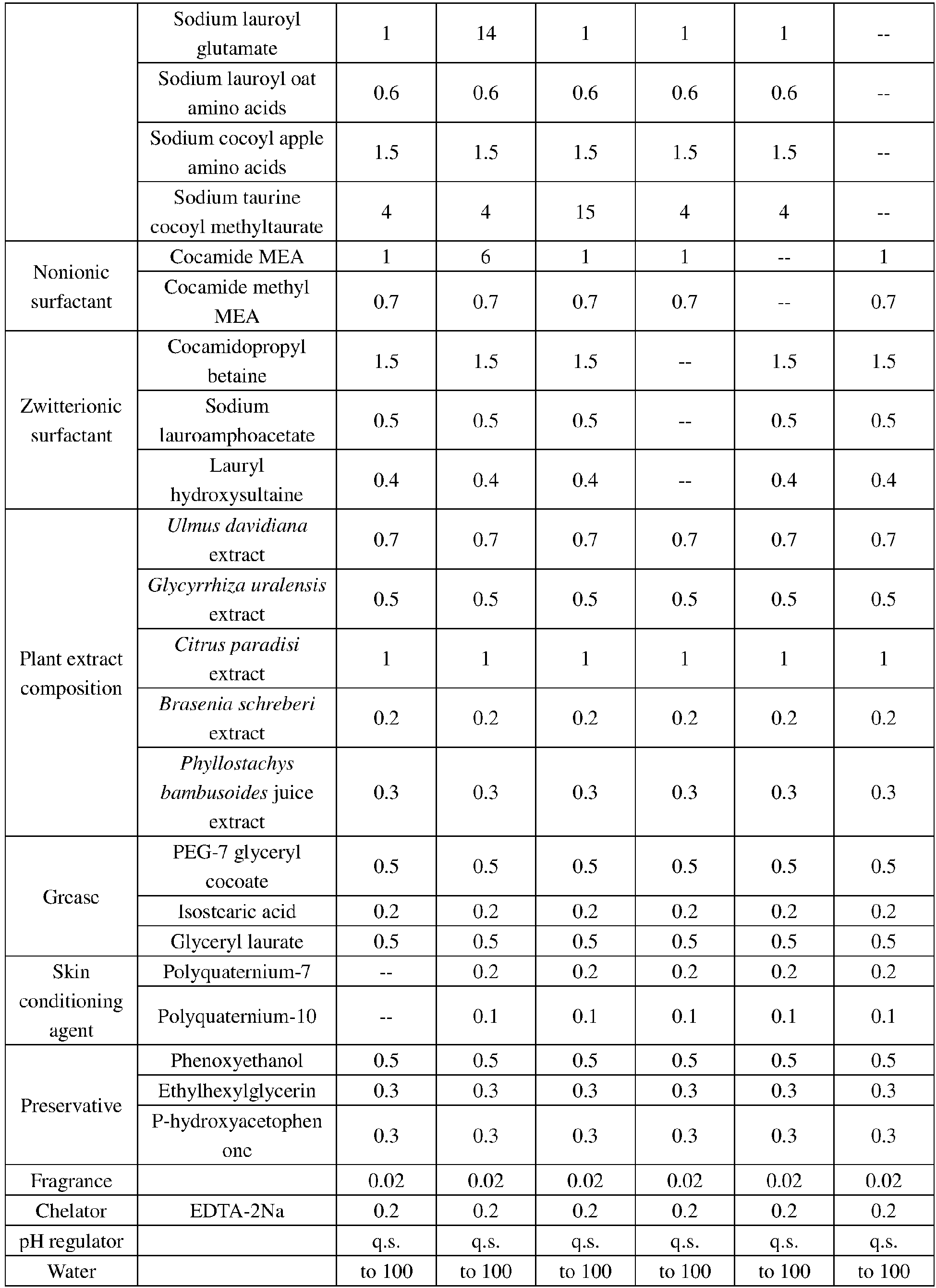

The shower gels of Comparative examples 1 to 13 were prepared by the same method as that of the shower gels of Examples 1 to 3.

The shower gels prepared in Comparative examples 1 to 13 were denoted as shower gels 4 to 16.

The difference from Example 1 is that Comparative example 1 did not contain the plant extract composition;
the difference from Example 1 is that Comparative example 2 did not contain an *Ulmus davidiana* extract;
the difference from Example 1 is that Comparative example 3 did not contain a *Glycyrrhiza uralensis* extract;
the difference from Example 1 is that Comparative example 4 did not contain a *Citrus paradisi* extract;
the difference from Example 1 is that Comparative example 5 did not contain a *Brasenia schreberi* extract;
the difference from Example 1 is that Comparative example 6 did not contain a *Phyllostachys bambusoides* juice extract;
the difference from Example 1 is that Comparative example 7 did not contain grease;
the difference from Example 1 is that Comparative example 8 did not contain a skin conditioning agent;
the difference from Example 1 is that the total amount of surfactants contained in Comparative example 9 exceeded the range;
the difference from Example 1 is that the total amount of surfactants contained in Comparative example 10 exceeded the range;
the difference from Example 1 is that Comparative example 11 did not contain an ampholytic surfactant;
the difference from Example 1 is that Comparative example 12 did not contain a nonionic surfactant; and
the difference from Example 1 is that Comparative example 13 did not contain an anionic surfactant.

### Efficacy Test

### Human patch test

1. Products to be tested: the shower gels of Examples 1 to 3, and the shower gels of Comparative examples 1 to 13;
2. Subjects: a total of 120 subjects including 70 males and 50 females of 20-55 years old who met the voluntary inclusion criteria for subjects;
3. Test method: an occlusive test method was adopted, the products to be tested were placed onto a suitable patch, the patch was applied to a curved side of an arm of a subject and gently pressed with a palm to be uniformly applied to the skin for 24 h; and the products were not applied to subjects in a blank control group;
4. Result observation: the patches were removed after 24 hours, skin reactions were observed after 0.5 h, 24 h, and 48 h, respectively, and reaction results were recorded. Grades of adverse skin reactions are shown in Table 3, and test results are shown in Table 4.

**Table 3 Standards for grading skin reactions in occlusive skin patch test**

| Grade | Skin reaction |
|---|---|
| 0 | Negative reaction |
| 1 | Doubtful reaction, faint erythema only |
| 2 | Weak positive reaction (erythema reaction): erythema, infiltration, edema, and possible papules |
| 3 | Strong positive reaction (vesicle reaction): erythema, infiltration, edema, and papules; and the reaction may extend out of the testing area: |
| 4 | Extreme positive reaction (coalescing vesicle reaction): intense erythema, severe infiltration, edema, and coalescing vesicles; and the reaction extends out of the testing area |

**Table 4 Results of skin patch test on human**

| Groups | Number of subjects | Observation time (h) | Number of subjects having different skin reactions in patch test | | | | |
|---|---|---|---|---|---|---|---|
| | | | 0 | 1 | 2 | 3 | 4 |
| Blank control group | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Example 1 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Example 2 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Example 3 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 1 | 60 | 0.5 | 59 | 1 | 0 | 0 | 0 |
| | | 24 | 59 | 1 | 0 | 0 | 0 |
| | | 48 | 60 | 0 | 0 | 0 | 0 |
| Compara tive example 2 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 3 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 4 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 5 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 6 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 7 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 8 | 60 | 0.5 | 60 | 0 | 0 | 0 | 0 |
| | | 24 | 60 | 0 | 0 | 0 | 0 |
| | | 48 | 60 | 0 | 0 | 0 | 0 |
| Compara tive example 9 | 60 | 0.5 | 58 | 1 | 1 | 0 | 0 |
| | | 24 | 60 | 0 | 0 | 0 | 0 |
| | | 48 | 60 | 0 | 0 | 0 | 0 |
| Compara tive example 10 | 60 | 0.5 | 60 | 0 | 0 | 0 | 0 |
| | | 24 | 60 | 0 | 0 | 0 | 0 |
| | | 48 | 59 | 1 | 0 | 0 | 0 |
| Compara tive example 11 | 60 | 0.5 | 60 | 0 | 0 | 0 | 0 |
| | | 24 | 59 | 1 | 0 | 0 | 0 |
| | | 48 | 60 | 0 | 0 | 0 | 0 |
| Compara tive example 12 | 60 | 0.5 | 59 | 1 | 0 | 0 | 0 |
| | | 24 | 60 | 0 | 0 | 0 | 0 |
| | | 48 | 60 | 0 | 0 | 0 | 0 |
| Compara tive example 13 | 60 | 0.5 | 60 | 0 | 0 | 0 | 0 |
| | | 24 | 60 | 0 | 0 | 0 | 0 |
| | | 48 | 60 | 0 | 0 | 0 | 0 |

The results show that among the 120 subjects, 0 subject of Example 1, Example 2, Example 3, Comparative example 2, Comparative example 3, Comparative example 4, Comparative example 5, Comparative example 6, Comparative example 7, Comparative example 8, Comparative example 9, Comparative example 10, Comparative example 11, Comparative example 12, and Comparative example 13 has adverse skin reactions, 2 subjects of Comparative example 1 have Grade 1 adverse reactions, 1 subject of Comparative example 9 has a Grade 1 adverse reaction, 1 subject of Comparative example 9 has a Grade 2 adverse reaction, 1 subject of Comparative example 10 has a Grade 1 adverse reaction, 1 subject of Comparative example 11 has a Grade 1 adverse reaction, and 1 subject of Comparative example 12 has a Grade 1 adverse reaction.

It is found through the comparison of Example 1, Example 2, Example 3, and Comparative example 1 that 0 subject of Example 1, Example 2, and Example 3 has adverse reactions, and 2 subject of Comparative example 1 have Grade 1 adverse reactions, which indicates that the shower gels containing the plant compositions are milder.

It is found through the comparison of Example 1, Example 2, Example 3, and Comparative example 9, Comparative example 10 that 0 subject of Example 1, Example 2, and Example 3 has adverse reactions, 1 subject of Comparative example 9 has a Grade 1 adverse reactions, 1 subject of Comparative example 9 has a Grade 2 adverse reaction, and 1 subject of Comparative example 10 has a Grade 1 adverse reaction, which indicates that the shower gels containing excessive surfactants will cause irritation to the skin.

It is found through the comparison of Example 1, Example 2, Example 3, and Comparative example 11, Comparative example 12 that 0 subjects of Example 1, Example 2, and Example 3 has adverse reactions, 1 subject of Comparative example 11 has a Grade 1 adverse reaction, and 1 subject of Comparative example 12 has a Grade 1 adverse reaction, which indicates that the shower gels containing zwitterionic surfactants and nonionic surfactants can reduce irritation.

The above results show that the shower gels of Examples 1 to 3 of the present disclosure are safe and have no irritation, and such an anti-dry and anti-sensitivity shower gel is safe and mild to the skin.

### Performance test

### Preservative efficacy challenge test

A preservative efficacy challenge test was performed according to the method described in *European Pharmacopoeia* (EP 7.0), and test results are shown in Table 5.

**Table 5 Preservative efficacy challenge test**

| | Microorganis ms | Inoculum concentrati on (cfu/g) | Micro-orga nisms concentrati on (cfu/g) after 7 days | Micro-orga nisms concentrati on (cfu/g) after 7 days | Micro-orga nisms concentrati on (cfu/g) after 7 days | Micro-orga nisms concentrati on (cfu/g) after 7 days |
|---|---|---|---|---|---|---|
| Example 1 | Bacteri a | 6.1×10⁶ | <10 | <10 | <10 | <10 |
| | Fungi | 5.7×10⁵ | <10 | <10 | <10 | <10 |
| | Molds | 4.0×10⁵ | <10 | <10 | <10 | <10 |
| | Yeasts | 2.7×10⁵ | <10 | <10 | <10 | <10 |
| Example 2 | Bacteri a | 6.1×10⁶ | <10 | <10 | <10 | <10 |
| | Fungi | 5.7×10⁵ | <10 | <10 | <10 | <10 |
| | Molds | 4.0×10⁵ | <10 | <10 | <10 | <10 |
| | Yeasts | 2.7×10⁵ | <10 | <10 | <10 | <10 |
| Example 3 | Bacteri a | 6.1×10⁶ | <10 | <10 | <10 | <10 |
| | Fungi | 5.7×10⁵ | <10 | <10 | <10 | <10 |
| | Molds | 4.0×10⁵ | <10 | <10 | <10 | <10 |
| | Yeasts | 2.7×10⁵ | <10 | <10 | <10 | <10 |

It can be seen from Table 3 that the shower gels of Examples 1 to 3 pass the preservative efficacy challenge test. In the testing process, microbial contamination in the production and use process of cosmetics was simulated to avoid possible harm to consumers when using the products, and the results show that the amino acid shower gels are safe.

### Human sensory evaluation

340 volunteers were recruited and randomly grouped according to 20 volunteers in each group, and after continuously using the shower gels of various groups for 8 weeks, they scored according to their feeling of use. The scoring criteria adopted a 7-point system, with 1 being the worst and 7 being the best. The higher the score, the better. Final results are shown in Table 6.

**Table 6**

| Evaluation item | appearance preference of the shower gel | Foam density of the shower gel | Whether the shower gel is easy to rinse after use | Moisture retention of the skin after use of the shower gel | Improvement of the scaly skin after use of the shower gel | Whether the antimicrobial activity is good, and whether the skin is itchy after use | Overall satisfaction with shower gel |
|---|---|---|---|---|---|---|---|
| Example 1 | 6.4 | 5.5 | 6.2 | 5.9 | 5.8 | 6.4 | 5.9 |
| Example 2 | 6.0 | 6.0 | 6.3 | 5.5 | 5.7 | 6.2 | 5.9 |
| Example 3 | 5.9 | 5.7 | 5.9 | 5.7 | 5.8 | 6.0 | 6.3 |
| Comparativ e example 1 | 4.8 | 4.8 | 4.5 | 3.9 | 2.9 | 4.3 | 4.9 |
| Comparativ e example 2 | 3.6 | 4.7 | 4.6 | 3.7 | 3.0 | 3.5 | 5.6 |
| Comparativ e example 3 | 5.0 | 4.9 | 5.0 | 5.2 | 2.5 | 3.0 | 5.3 |
| Comparativ e example 4 | 3.6 | 5.1 | 5.0 | 4.3 | 2.3 | 3.2 | 3.0 |
| Comparativ e example 5 | 4.0 | 5.0 | 4.9 | 3.5 | 3.7 | 2.9 | 4.8 |
| Comparativ e example 6 | 3.9 | 4.6 | 4.7 | 3.6 | 3.5 | 2.3 | 4.6 |
| Comparativ e example 7 | 3.6 | 5.8 | 5.6 | 2.2 | 2.9 | 3.1 | 3.8 |
| Comparativ e example 8 | 4.6 | 5.0 | 4.5 | 4.3 | 4.0 | 3.8 | 4.1 |
| Comparativ e example 9 | 3.1 | 5.1 | 5.5 | 3.1 | 3.1 | 3.2 | 2.4 |
| Comparativ e example 10 | 3.6 | 6.2 | 5.7 | 3.4 | 3.3 | 3.7 | 2.7 |
| Comparativ e example 11 | 3.4 | 3.2 | 5.8 | 2.9 | 3.0 | 3.8 | 2.9 |
| Comparativ e example 12 | 3.2 | 3.4 | 5.5 | 2.8 | 3.2 | 3.6 | 3.3 |
| Comparativ e example 13 | 1.3 | 1.4 | 2.1 | 4.6 | 4.5 | 4.3 | 2.0 |
| Commercial ly available amino acid shower gel A | 5.6 | 4.5 | 4.2 | 3.9 | 3.3 | 3.7 | 4.4 |

It can be seen from Table 6 that the shower gel compositions of the present disclosure have relatively great advantages over the commercially available amino acid shower gel A in terms of easy rinsing after use, moisture retention of the skin, itching resistance, scaly skin improvement after use. They are generally favoured by the subjects.

Compared with Example 1, Comparative example 1, Comparative example 2, Comparative example 3, Comparative example 4, Comparative example 5, and Comparative example 6 which contained different plant extract compositions had lower scores in terms of itching resistance, scaly skin improvement, and consumer satisfaction after use.

Compared with Example 1, Comparative example 7 and Comparative example 8 without grease and skin conditioning agents had lower scores in terms of moisturizing after use.

Compared with Example 1, Comparative example 9 and Comparative example 10 which contained surfactants exceeding the recommended range made improvement in terms of foaming, but had lower scores in terms of scaly skin improvement, itching resistance, and overall satisfaction after use due to excessive degreasing.

Compared with Example 1, Comparative example 11 and Comparative example 12 without ampholytic surfactants and nonionic surfactants had lower scores in terms of foam density, appearance preference of shower gel, and overall satisfaction with shower gel.

Compared with Example 1, Comparative example 13 without anionic surfactants had lower scores in terms of foam density, cleanliness of the skin, and overall satisfaction after use.

The above results show that the amino acid shower gels of the present disclosure have good feeling of use, and have a moisturizing effect, an anti-itching effect, a scaly improvement effect, etc. Furthermore, various components in the formulas of the shower gels of the present disclosure achieve a synergistic effect, and thus the overall use effect is better.

The preferred specific embodiments of the present disclosure are described in detail above, which are obtained by the inventors in many tests after spending a lot of manpower, financial resources, and time. It should be understood that ordinary of skill in the art can make many modifications and changes according to the concept of the present disclosure without any creative efforts.

## Claims

1. A plant extract composition, **characterized by** comprising the following components in percentage by weight: 0.1-30% of *Ulmus davidiana* extract, 0.05-45% of *Glycyrrhiza uralensis* extract, 0.01-26% of *Citrus paradisi* extract, 0.05-20% of *Brasenia schreberi* extract, and 0.02-35% of *Phyllostachys bambusoides* juice extract.

2. A composition, **characterized by** comprising the following components in percentage by weight: 10-40% of anionic surfactant, 1-12% of nonionic surfactant, 1-28% of zwitterionic surfactant, 0.5-40% of plant extract composition according to claim 1, 0.5-35% of grease, and 0.1-6% of skin conditioning agent.

3. The composition according to claim 2, **characterized in that** the anionic surfactant is selected from at least one of sodium lauroyl sarcosinate, sodium lauroyl alaninate, sodium lauroyl glutamate, sodium lauroyl oat amino acids, sodium cocoyl apple amino acids, and sodium taurine cocoyl methyltaurate;
preferably, the anionic surfactant is selected from the following components in percentage by weight: 3-30%, and preferably 6-20% of sodium lauroyl sarcosinate; 0.5-10%, and preferably 1-7% of sodium lauroyl alaninate; 0.1-9%, and preferably 0.5-6% of sodium lauroyl glutamate; 0.2-11%, and preferably 0.5-8% of sodium lauroyl oat amino acids; 0.5-12%, and preferably 1-5% of sodium cocoyl apple amino acids; and 1-12%, and preferably 3-8% of sodium taurine cocoyl methyltaurate.

4. The composition according to claim 2 or 3, **characterized in that** the nonionic surfactant is selected from at least one of cocamide MEA and cocamide methyl MEA;
preferably, the nonionic surfactant is selected from the following components in percentage by weight: 0.1-5%, and preferably 0.5-2% of cocamide MEA; and 0.2-6%, and preferably 0.6-3% of cocamide methyl MEA.

5. The composition according to any one of claims 2 to 4, **characterized in that** the zwitterionic surfactant is selected from at least one of cocamidopropyl betaine, sodium lauroamphoacetate, and lauryl hydroxysultaine;
preferably, the zwitterionic surfactant is selected from the following components in percentage by weight: 0.5-25%, and preferably 1-20% of cocamidopropyl betaine; 0.3-13%, and preferably 0.4-12% of sodium lauroamphoacetate; and 0.1-23%, and preferably 0.3-9% of lauryl hydroxysultaine.

6. The composition according to any one of claims 2 to 5, **characterized in that** the plant extract composition is selected from the following components in percentage by weight: 0.1-20%, and preferably 0.5-15% of *Ulmus davidiana* extract; 0.1-16%, and preferably 0.3-15% of *Glycyrrhiza uralensis* extract; 0.2-8%, and preferably 0.5-6% of *Citrus paradisi* extract; 0.03-17%, and preferably 0.1-11% of *Brasenia schreberi* extract, and 0.03-5%, and preferably 0.2-4% of *Phyllostachys bambusoides* juice extract.

7. The composition according to any one of claims 2 to 6, **characterized in that** the grease is selected from at least one of PEG-7 glyceryl cocoate, isostearic acid, and glyceryl laurate;
preferably, the grease is selected from the following components in percentage by weight: 0.1-4%, and preferably 0.2-1% of PEG-7 glyceryl cocoate; 0.1-2%, and preferably 0.2-1% of isostearic acid; and 0.1-5%, and preferably 0.2-3% of glyceryl laurate.

8. The composition according to any one of claims 2 to 7, **characterized in that** the skin conditioning agent is selected from at least one of polyquaternium-7 and polyquaternium-10;
preferably, the skin conditioning agent is selected from the following components in percentage by weight: 0.05-1%, and preferably 0.1-0.6% of polyquaternium-7; and 0.01-2%, and preferably 0.1-0.6% of polyquaternium-10.

9. The composition according to any one of claims 2 to 8, **characterized by** further comprising one or more of a preservative, a chelator, a pH regulator, a fragrance, and water;
preferably, the preservative is selected from at least one of phenoxyethanol, ethylhexylglycerin, and p-hydroxyacetophenone;
preferably, the preservative is selected from the following components in percentage by weight: 0.2-1%, and preferably 0.3-0.6% of phenoxyethanol; 0.1-1%, and preferably 0.2-0.6% of ethylhexylglycerin; and 0.1-1%, and preferably 0.2-0.5% of p- hydroxyacetophenone;
preferably, the chelator is selected from EDTA-2Na; and preferably, the chelator has a weight percentage of 0.1-0.6%, and preferably 0.2-0.4%;
preferably, the pH regulator is selected from at least one of citric acid, sodium citrate, aminomethyl propanol, and triethanolamine;
preferably, the pH regulator is selected from citric acid; and further preferably, the citric acid has a weight percentage of 0.01-8%; and
preferably, the fragrance has a weight percentage of 0.02%.

10. A preparation method of the composition according to claim 9, **characterized by** comprising the following steps:
at step 1), adding an anionic surfactant and water into a reaction kettle, stirring, and heating to 75-80°C;
at step 2), adding a zwitterionic surfactant, and stirring uniformly;
at step 3), further adding a nonionic surfactant and a chelator, stirring uniformly, keeping the temperature for 20-30 min, and cooling;
at step 4), cooling to 45°C, adding a plant composition, a preservative, and grease, and stirring uniformly;
at step 5), adding a skin conditioning agent and a fragrance, and regulating pH to 5.5-7.3; and
at step 6), cooling to 37°C, and filtering.
